# EUROPEAN PATENT SPECIFICATION

(11) **EP 1 981 572 B1**
(45) Date of publication and mention of the grant of the patent: **21.11.2012**
(21) Application number: 07706125.7
(22) Date of filing: 08.02.2007
(51) Int. Cl.: A61M 15/00, A61K 38/57, A61P 11/00

(54) **PULMONARY DELIVERY OF ALPHA-I PROTEINASE INHIBITOR**
PULMONALE ABGABE VON ALPHA-I PROTEINASE-HEMMER
ADMINISTRATION PULMONAIRE D'UN INHIBITEUR DE LA PROTÉINASE ALPHA-1

(30) Priority: 09.02.2006 US 771465 P; 16.02.2006 US 773654 P
(43) Date of publication of application: 22.10.2008
(73) Proprietor: Kamada Ltd., 74140 Ness-Ziona (IL); PARI Pharma GmbH, 82319 Starnberg (DE)
(72) Inventor: BAUER, Shabtai, IL-93702 Jerusalem (IL); KELLER, Manfred, D-81379 Munich (DE); KNOCH, Martin, D-82335 Berg (DE)
(74) Representative: Becker Kurig Straus
(86) International application number: PCT/IL2007/000182
(87) International publication number: WO 2007/091267

(56) References cited:
- WO-A-2004/052436
- WO-A-2005/048985
- DE-C1- 19 953 317
- US-A- 5 618 786
- HUBBARD R C ET AL: "ANTI-NEUTROPHIL-ELASTASE DEFENSES OF THE LOWER RESPIRATORY TRACT IN ALPHA-1 ANTITRYPSIN DEFICIENCY DIRECTLY AUGMENTED WITH AN AEROSOL OF ALPHA-1 ANTITRYPSIN" ANNALS OF INTERNAL MEDICINE, vol. 111, no. 3, 1989, pages 206-212, XP002448572 ISSN: 0003-4819
- DATABASE BIOSIS [Online] BIOSCIENCES INFORMATION SERVICE, PHILADELPHIA, PA, US; January 2002 (2002-01), BRAND P ET AL: "Alveolar deposition of monodisperse aerosol particles in the lung of patients with chronic obstructive pulmonary disease" XP002449365 Database accession no. PREV200200118466 & EXPERIMENTAL LUNG RESEARCH, vol. 28, no. 1, January 2002 (2002-01), pages 39-54, ISSN: 0190-2148

## Description

### FIELD OF THE INVENTION

The present invention relates to a system and methods of treating pulmonary diseases comprising the administration of a ready-to-use liquid composition comprising alpha-1 proteinase inhibitor (API) in aerosol form in a nebulizer. The API compositions are adapted to optimize the delivery of the active ingredient to the lungs.

### BACKGROUND OF THE INVENTION

### Alpha-1 Proteinase Inhibitor and Lung Disease

Alpha-1 proteinase inhibitor (API), also known as Alpha-1-antitrypsin (A1AT, AAT) and Serine protease inhibitor, is a plasma-derived protein belonging to the family of serine proteinase inhibitors. It is a glycoprotein having an average molecular weight of 50,600 daltons, produced by the liver and secreted into the circulatory system. The protein is a single polypeptide chain, to which several oligosaccharide units are covalently bound. API has a role in controlling tissue destruction by endogenous serine proteinases, and is the most prevalent serine proteinase inhibitor in blood plasma. API inhibits, *inter alia,* trypsin, chymotrypsin, various types of elastases, skin collagenase, renin, urokinase and proteases of polymorphonuclear lymphocytes.

API is currently used therapeutically for the treatment of pulmonary emphysema in patients who have a genetic API deficiency, also known as Alpha-1 Antitrypsin Deficiency or Congenital Emphysema. Purified API has been approved for replacement therapy (also known as "augmentation therapy") in these patients. The endogenous role of API is to regulate the activity of neutrophil elastase, which breaks down foreign proteins present in the lung. In the absence of sufficient quantities of API, the elastase breaks down lung tissue, which over time results in chronic lung tissue damage and emphysema.

API has also been proposed as a treatment for cystic fibrosis (CF) patients who suffer from recurrent endobronchial infections and sinusitis. The major cause of morbidity and mortality among CF patients is lung diseases. CF patients carry a mutation in the CFTR gene, resulting in a malfunctioning CTFR protein, defective water and salt transport and the ensuing thick secretions in the lung.

The membrane defect caused by the CFTR mutation leads to chronic lung inflammation and infection. In normal individuals, elastase secreted by neutrophils in response to infection is neutralized by API. API is known to penetrate into pulmonary tissue and exert its activity within this tissue. In patients with CF, however, the unregulated inflammatory response overwhelms the normal protease (elastase)/antiprotease (API) balance. The abnormal cycle is destructively self-perpetuating and leads to the accumulation of elastase in the lung and ultimately to tissue damage, destruction of the lung architecture, severe pulmonary dysfunction and, ultimately, death. Supplemental API may reduce the deleterious effects associated with excessive amounts of elastase. International application WO 2005/027821 to one of the applicants of the present invention teaches a novel composition of purified, stable, active alpha-1 proteinase inhibitor (API) for intravenous administration and inhalation, and process for its preparation. That application teaches an aerosol formulation comprising about 10% to about 20% API.

US-A-5618 786 teaches the treatment of pulmonary diseases by ultrasonic nebulizers containing API.

API is currently administered intravenously. For example, the Aralast^{®}, Zemaira^{®} and Prolastin^{®} brands of human Alpha-1-Proteinase Inhibitor are intravenous formulations indicated for augmentation therapy in patients having congenital deficiency of API with clinically evident emphysema. An API formulation for efficient administration in inhalation is highly desired, and not yet commercially available due to problems in achieving suitable quantity, dispersion and activity of the protein. International Application WO2005048985 discloses compositions comprising API, which further comprise a stabilizing carbohydrate, a surfactant and an antioxidant to stabilize the API for use as a therapeutic, wherein the composition is preferably formulated to be administered by inhalation.

### Pulmonary Delivery of Pharmaceutical Compositions

International application WO 01/34232 to some of the applicants of the present invention discloses an inhalation nebulizer providing an increased amount of aerosol during inhalation while minimizing both aerosol losses during exhalation and the residual drug in the nebulizer reservoir. The nebulizer includes an aerosol generator that atomizes the liquid through a vibrating diaphragm into particle sizes that are efficiently delivered to the lungs. This nebulizer is currently commercialized under the trade name eFlow^{®}. Classic jet and ultrasonic nebulizers have the disadvantage of potentially denaturizing the active agent by high shear forces (jet and ultrasonic nebulizers) and temperature increase (ultrasonic nebulizers). eFlow^{®} incorporates a "gentle" aerosolization mechanism that minimizes exposure of the drug to shear stresses by reducing the shear stresses and the residence time in the shear fields and does not heat the liquid formulation. International Applications WO 03/026832; WO 2004/014569; WO 2004/052436 and U.S. Patent 5,518,179 disclose further aspects of the eFlow^{®} technology. The use of the nebulizer disclosed in WO 01/34232 is exemplified with a salbutamol/sulfate solution and a budesonide suspension. Alpha anti-trypsin is listed as a potential active agent for use with that nebulizer.

U.S. Patent No. 6,655,379 discloses a method and device for the pulmonary delivery of an active agent formulation where inspiratory flow rate of the active agent formulation is less than 17 liters/min. The active agent formulation may be provided in dry powder, in nebulized form, or in the form of aerosolized particles in admixture with a propellant. That invention is exemplified in conjugation with inhalable insulin powder.

Therefore, there remains an unmet need for inhalable aerosols for convenient and efficient pulmonary delivery of API in an active form.

### SUMMARY OF THE INVENTION

The present invention relates to a system and methods of treating pulmonary diseases in a subject in need thereof. The system and methods provide administration to the subject of a composition comprising API in the form of an aerosol. The aerosol is produced by nebulizing the API composition in an electronic nebulizer. Particularly, the nebulizer is eFlow^{®} electronic nebulizer, as disclosed in International Application WO 01/34232, and the API composition is a ready-to-use solution comprising highly pure, active API, as disclosed in International Application WO 2005/027821.

The present invention discloses an inhalation system providing high amounts of active API within the desired location of the lung tissues, thus enabling an efficient treatment of pulmonary diseases.

The invention is based in part on the unexpected discovery that pulmonary delivery of a composition comprising less than 10% (weight/volume) API nebulized in the eFlow^{®} nebulizer is efficient and effective. The API composition is preferably a ready-to-use sterilized liquid composition comprising highly pure, active API. The present invention now shows that the activity of API after nebulization remains at above 90% of the activity before nebulization, and, due to the high purity of the composition there is no evidence of dimers, oligomers or aggregates formed in the API aerosol as a result of the nebulization process. Furthermore, the majority of aerosol droplets is in a size range of less than 5 µm and more specifically between 1 and 4 µm enabling maximal uptake in the peripheral lung regions. Advantageously, using the system of the present invention reduces inhalation time due to a high liquid output rate and efficient delivery of the active API to the lung.

In addition, the high purity and high activity of the API in combination with the unique features of the nebulizer, including "gentle" vibrating membrane aerosolization principle which results in low shear stresses on protein molecules, ensures high efficacy and safety. The ready-to-use stable liquid formulation, comprising highly pure, active API is convenient and provides improved dosing accuracy compared to lyophilized products.

Despite considerable development efforts over the past decade, other forms of inhaled API have not yet been shown to be successful. The combination of an efficient and fast delivery device with highly pure, stable and active API in ready-to-use liquid formulation packed in predetermined dose vials further provides improved acceptance and compliance by patients compared to intravenous administration and previous attempts for inhaled API therapies.

Accordingly, in one aspect the present invention provides a system for the treatment of pulmonary diseases comprising:
a) a pharmaceutical composition comprising a purified, stable, active alpha-1 proteinase inhibitor (API) produced by the liver and secreted into circulatory system in a form of a ready to use sterile solution wherein the purified, stable alpha-1 proteinase inhibitor is at least 90% pure ; and
b) an inhalation nebulizer comprising
   i) an aerosol generator comprising: a liquid storage container comprising the liquid pharmaceutical composition; a diaphragm having a first side and an opposite second side, the diaphragm having a plurality of openings extending therethrough from the first side to the second side, where the first side is connected to the liquid storage container such that the liquid filled into the liquid storage container comes into contact with the first side of the diaphragm; and a vibration generator capable of vibrating the diaphragm so that the liquid filled into the liquid storage container is atomized on the second side of the diaphragm through the openings of the diaphragm;
   ii) a mixing chamber into which the aerosol generator expels said aerosol, the mixing chamber in contact with the second side of the diaphragm;
   iii) an inhalation valve that is open to allow an inflow of ambient air into the mixing chamber during an inhalation phase and is closed to prevent escape of said aerosol from the mixing chamber during an exhalation phase; and
   iv) an exhalation valve that is open to allow the discharge of the respiratory air of a patient into the surroundings during the exhalation phase and is closed to prevent the inflow of ambient air during the inhalation phase;
wherein the pharmaceutical composition is nebulized by the inhalation nebulizer to form an aerosol composition and wherein at least 90% of the pure, nebulized alpha-1 proteinase inhibitor is units active form.

According to certain embodiments, the liquid composition is packed in a pre-sterilised unit dose.

In one embodiment the API in the pharmaceutical composition is purified from a partially purified mixture of proteins by a process comprising chromatography on a plurality of ion exchange resins, as disclosed in International Application WO 2005/027821 to one of the Applicants of the present invention. As disclosed in that invention, the API in the pharmaceutical composition is preferably purified from a partially purified mixture of proteins by a process comprising chromatography on at least two anion exchange resins and at least one cation exchange resin.

In one embodiment the concentration of API in the pharmaceutical composition is less than 10% (weight per volume; w/v). In certain embodiments the concentration of API is between about 1% to about 5%, preferably about 2%. In another embodiment the pH of the pharmaceutical composition is in the range of 6.5-7.5. In yet further embodiment, the pharmaceutical composition is devoid of a protein stabilizer.

The purified AAT is at least 90% pure. According to preferred embodiments the purified AAT is at least 95%, more preferably at least 99% pure. At least 90% of the API is in its active form.

In another embodiment the mass median diameter of the aerosol droplet size distribution is less than about 5 µm; preferably the mass median diameter of the aerosol droplets is between about 1 µm and about 4 µm, more preferably between about 2.0 µm and about 3.5 µm and most preferably between about 2.5 µm to about 3.3 µm. Furthermore, a very narrow droplet size distribution have been found (reflected by a remarkably low geometric standard deviations (GSD)) allowing for targeted delivery of the drug to the deep lungs. According to one embodiment, the GSD is lower than about 2.0. According to another embodiment, the GSD is lower than about 1.7.

According to another embodiment, at least a fraction of 50%, preferably 60%, more preferably 70% and more of the loaded nominal dose of API is dissolved in droplets having a diameter from about 1.1 µm to about 4.7 µm.

According to another aspect, the present invention provides the use of a pharmaceutical composition comprising a purified, stable, active alpha-1 proteinase inhibitor (API) produced by the liver and secreted into the circulatory system in a form of a ready to use sterile solution, wherein the stable alpha-1 protainase inhibitor is at least 90% pure; and b) an inhalation nebulizer comprising
i) an aerosol generator comprising: a liquid storage container comprising the liquid pharmaceutical composition; a diaphragm having a first side and an opposite second side, the diaphragm having a plurality of openings extending therethrough from the first side to the second side, where the first side is connected to the liquid storage container such that the liquid filled into the liquid storage container comes into contact with the first side of the diaphragm; and a vibration generator capable of vibrating the diaphragm so that the liquid filled into the liquid storage container is atomized on the second side of the diaphragm through the openings of the diaphragm;
ii) a mixing chamber into which the aerosol generator expels said aerosol, the mixing chamber in contact with the second side of the diaphragm;
iii) an inhalation valve that is open to allow an inflow of ambient air into the mixing chamber during an inhalation phase and is closed to prevent escape of said aerosol from the mixing chamber during an exhalation phase; and
iv) an exhalation valve that is open to allow the discharge of the respiratory air of a patient into the surroundings during the exhalation phase and is closed to prevent the inflow of ambient air during the inhalation phase;
for the preparation of a system comprising said pharmaceutical composition and said nebulizer wherein the pharmaceutical composition is nebulized by the inhalation nebulizer to form an aerosol composition, for administering to a subject a therapeutically effective amount of the aerosol composition comprising alpha-1 proteinase inhibitor, wherein at, least 90% of the pure nebulized alpha-1 proteinase inhibitor is in its active form.

In one embodiment, at least 50% of the loaded nominal dose of API can be delivered to the subject, preferably 60% and more preferably 70% or more of the API is delivered to the subject. According to one currently preferred embodiment, the API is absorbed by lung tissues of the subject.

According to certain embodiments, the pulmonary disease or disorder is selected from the group consisting of emphysema, including inherited emphysema; chronic obstructive pulmonary disease (COPD); bronchiectasis (chronic dilatation of the bronchial tubes); fibrotic lung diseases or disorders including cystic fibrosis, interstitial pulmonary fibrosis and sarcoidosis; tuberculosis; and pulmonary diseases secondary to HIV.

API is required to balance the excess concentration of neutrophil elastase present during episodes of inflammation and stress that occur in pulmonary diseases at the phase of exacerbation. The high amounts of active API that can be delivered to the lung tissue by the system and method of the present invention makes it highly suitable to treat pulmonary diseases at the phase of exacerbation.

According to certain embodiments, the system and/or method is used for treating a pulmonary disease at a phase of acute exacerbation of the disease. According to one embodiment, the disease is selected from the group consisting of emphysema, chronic obstructive pulmonary disorder (COPD), bronchiectasis, other parenchymatic or fibrotic lung diseases including cystic fibrosis, interstitial pulmonary fibrosis and sarcoidosis, tuberculosis and pulmonary diseases secondary to HIV.

In one embodiment a subject is a human subject. The human subject may be an adult, a child or an infant.

These and other embodiments of the present invention will become apparent in conjunction with the description and claims that follow.

### BRIEF DESCRIPTION OF THE FIGURES

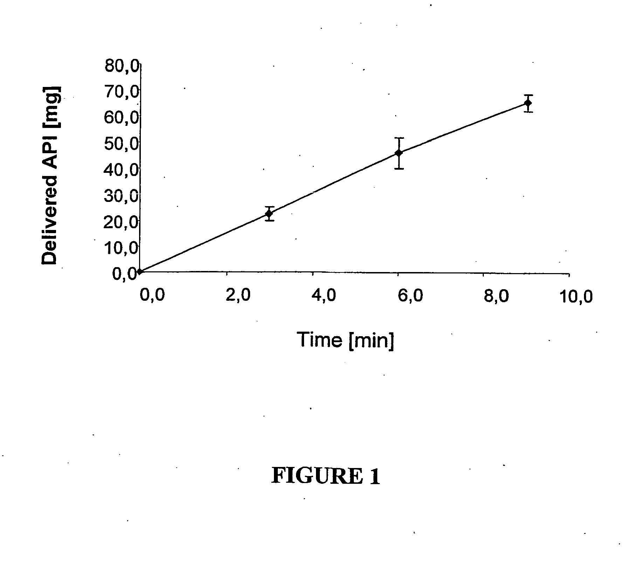

FIG. 1 shows the drug delivery profile after breath simulation with API.

### DETAILED DESCRIPTION OF THE INVENTION

The present invention discloses a system and methods of treating a pulmonary disease in a subject in need thereof, comprising the administration of a ready to use liquid pharmaceutical composition comprising a purified, stable, active alpha-1 proteinase inhibitor (API) in the form of an aerosol produced by nebulizing the API composition, particularly nebulizing the API composition in an eFlow^{®} nebulizer.

The route of inhalation for the treatment of respiratory diseases has several advantages over other routes of administration, specifically IV administration: Inhalation delivery is directed to the target site, such that there is negligible systemic absorption and side effects are minimized; it requires lower therapeutic doses, and thus there is a greater product availability; it provides quick relief of symptoms and expected good tolerance; it is more convenient form for patients thus better compliance is expected; and it reduces treatment costs as a result of efficient utilization of an expensive drug using stable, purified API with a highly efficient nebulizer such as the eFlow^{®}.

In principle a therapeutically effective system for pulmonary delivery of aerosolized alpha-1 proteinase inhibitor will exhibit the following properties:
1. The aerosol droplets retain high activity of alpha-1 proteinase inhibitor: an activity of greater than 90% is desired;
2. The nebulization does not promote API dimerization, oligomerization or aggregation;
3. Aerosol median droplet size of less than 5 µm;
4. Respirable fraction of at least 80%;
5. A very low ballistic momentum characterised in that less than 2% of the drug dissolved or dispersed in the aerosol droplets can be trapped in an induction port (i.e. artificial oropharynx) of a cascade impactor;
6. Short, single treatment time of about 10 minutes or less.

The present invention provides a system comprising pure API delivered via the eFlow® nebulizer exhibiting, *inter alia,* the aforementioned advantageous properties. Additionally, the present invention further provides a liquid composition comprising API at a concentration of less than 10% w/v, preferably a ready-to use liquid composition.

The high activity and purity of the API composition in combination with the gentle vibrating membrane aerosolization mechanism ensures delivery of highly active API. The ready-to-use stable liquid formulation is convenient to use and obviates the problem of dose preparation and dosing accuracy.

### Definitions

In order that this invention may be better understood, the following terms and definitions are herein provided.

As used herein, the term "Alpha-1 Proteinase Inhibitor" (API), also known as "alpha-1 antitrypsin" (AAT), refers to a glycoprotein produced by the liver and secreted into the circulatory system. API belongs to the Serine Proteinase Inhibitor (Serpin) family of proteolytic inhibitors. This glycoprotein consists of a single polypeptide chain containing one cysteine residue and 12-13% carbohydrates of the total molecular weight. API has three N-glycosylation sites at asparagine residues 46, 83 and 247, which are occupied by mixtures of complex bi- and triantennary glycans. This gives rise to multiple API isoforms, having isoelectric point in the range of 4.0 to 5.0. The glycan monosaccharides include N-acetylglucosamine, mannose, galactose, fucose and sialic acid. API serves as a pseudo-substrate for elastase; elastase attacks the reactive center loop of the API molecule by cleaving the bond between methionine₃₅₈ - serine₃₅₉ residues to form an API-elastase complex.

The term "glycoprotein" as used herein refers to a protein or peptide covalently linked to a carbohydrate. The carbohydrate may be monomeric or composed of oligosaccharides.

The term "eFlow^{®}" nebulizer refers to the nebulizer disclosed in international application WO 01/34232. The term "inhalation nebulizer" refers to a nebulizer comprising the basic elements of the eFlow^{®} nebulizer and any equivalent nebulizer. The terms "pulmonary delivery" and "respiratory delivery" refer to delivery of API to a patient by inhalation through the mouth and into the lungs.

The terms "treat" and "treating" includes preventing, alleviating, ameliorating, halting, restraining, slowing or reversing the progression, or reducing the severity of pathological conditions described above. As such, these methods include both medical therapeutic (acute) and/or prophylactic (prevention) administration as appropriate.

The terms "cystic fibrosis" or "CF" refer to an inherited autosomal recessive disorder caused by mutations in the gene encoding the cystic fibrosis transmembrane conductance regulator (CFTR) chloride channel.

The term "emphysema" refers to a pathological condition of the lungs in which there is a decrease in respiratory function and often breathlessness due to an abnormal increase in the size of the air spaces, caused by irreversible expansion of the alveoli and/or by the destruction of alveolar walls by neutrophil elastase.

The term "COPD or "chronic obstructive pulmonary disease" refers to lung disease characterized by obstruction to airflow that interferes with normal breathing. COPD is the fourth leading cause of death in America, claiming the lives of 120,000 Americans in 2002. Smoking is the primary risk factor for COPD with approximately 80% to 90% of COPD deaths are caused by smoking.

The term "bronchiectasis" refers to a congenital or acquired disorder associated with abnormal bronchial dilatation with bronchial wall destruction and transmural inflammation. The most important functional finding of altered airway anatomy is severely impaired clearance of secretions from the bronchial tree.

"Tuberculosis" or "TB" refers to the disease caused by the *M*. *tuberculosis* bacterium. TB is an airborne, chronic bacterial infection.

As used herein, the term "exacerbation" refers to an increase in the severity of symptoms during a course of a disease which is mostly associated with a worsening of quality of life. Exacerbations are quite frequent in patients with chronic lung diseases and more specifically in API deficient patients. By definition, exacerbations are simply a worsening and/or increase in symptoms.

### Emphysema and API deficiency

Emphysema is a pathological condition of the lungs marked by an abnormal increase in the size of the air spaces, resulting in strenuous breathing and an increased susceptibility to infection. It can be caused by irreversible expansion of the alveoli or by the destruction of alveolar walls. Congenital emphysema, also known as Alpha-1 Antitrypsin (AAT) deficiency or inherited emphysema, is a genetic disorder that increases the risk of developing a variety of diseases including pulmonary emphysema and cirrhosis of the liver. It is caused by mutation in the gene coding for API, the body major serine proteinase inhibitor or serpin. This gene is located in the long arm of chromosome 14 of the human genome. The genetic deficiency can result in life-threatening liver disease in children and adults or in lung disease in adults. In its classic form, an inherited mutation of the API gene causes the build up of abnormal API within the hepatocytes of the liver. The liver is the major source of circulating API and this transport problem leads to low levels of API in the blood and tissue. Various mutations of the API gene exist, most of them associated with deficiency in circulating API. Genotypes that lead to the production of a protein that is dysfunctional as an elastase inhibitor and cause increased risk of emphysema, but are released at a normal level to the circulation have been also identified. It has been estimated that in the US only there are approximately 100,000 severely deficient individuals and about 25 million carriers of at least one deficient gene for API, and similar number have been suggested for the European population (Sandhaus 2004, supra). The classic proteinase pathogenesis model of congenital emphysema was based on failure of the body to neutralize elastase-related enzymatic activity, the elastase being released from polymorphonuclear leukocytes attracted to the lung alveoli during infections. Based on the nature of the triggering event and the cellular physiology of API as an elastase inhibitor it has been recently suggested that API is also functioning as an acute-phase reactant.

### Cystic Fibrosis

According to another embodiment, the method of the present invention is used for treating lung diseases and disorders associated with cystic fibrosis.

The membrane defect caused by the CFTR mutation leads to chronic lung inflammation and infection. Chronic lower respiratory infection provokes a persistent inflammatory response in the airway, resulting in chronic obstructive disease. As pulmonary reserves decrease, CF patients become prone to episodes of exacerbation, characterized by worsening symptoms of respiratory infection, particularly by *Pseudomonas aeruginosa,* accompanied by acute decline in lung function.

Loss of pulmonary function is a primary cause of death in patients suffering from cystic fibrosis. Patients with a Forced Expiratory Volume in one second (FEV1) below 30% of their predicted value have a 2-year mortality of greater than 50%. The current mortality rate is 1.2 deaths per 100 patients per year; the median survival is 32 years. Of the deaths in which a case was specified, 94% were due to cardiorespiratory failure. Respiratory failure is characterized by increasing dyspnea, hypoxemia and elevation of arterial PCO₂. During their lifetime, CF patients are restricted in their day-to-day activities due to reduced lung function and constant pulmonary infections as a result of their condition.

One of the major side effects of chronic infection associated with CF is the chronic presence of phagocytic neutrophils in the lungs in response to bacterial infections and the release of various chemoattractants. These leukocytes secrete elastase, which has the potential to destroy the elastic tissue of the lung. In addition, neutrophils of patients with CF have been shown to be in a state of increased responsiveness and tend to degranulate more readily, releasing tissue-destroying elastase. Thus, patients with CF appear to have a state of unregulated inflammatory response, which overwhelms the normal protease (elastase)/antiprotease (API) balance, leading to the accumulation of elastase in the lung and ultimately to tissue damage.

Previous studies have shown that much of the pulmonary damage in CF results from the presence of unneutralized elastase and other proteases. The abnormal cycle is destructively self-perpetuating and self-expanding: increased elastase leads to the recruitment of more neutrophils to the lung that in turn secrete additional proteases. This cycle further overwhelms the natural normal protease (elastase)/antiprotease balance leading to destruction of the lung architecture, severe pulmonary dysfunction and ultimately death. It has been suggested that supplement of additional API may reduce the deleterious effects associated with excessive amounts of elastase.

Preferably, API is administered to CF patients by the inhalation route. It has been previously demonstrated (McElvaney et al, 1991) that aerosolized alpha-anti-trypsin given to cystic fibrosis patients suppressed neutrophil elastase in the respiratory epithelial lining fluid (ELF), restored the anti-neutrophil elastase capacity in the ELF and reversed the inhibitory effect of the ELF on the ability of neutrophils to effectively combat *Pseudomonas* infections. Advantageously, aerosol compositions comprising active API can be readily produced using the system of the present invention.

### Bronchiectasis

Bronchiectasis is an abnormal dilation of the proximal medium-sized bronchi (>2 mm in diameter) caused by destruction of the muscular and elastic components of the bronchial walls. It can be congenital or acquired.

Congenital bronchiectasis usually affects infants and children and results from developmental arrest of the bronchial tree. The more commonly acquired forms occur in adults and older children and require an infectious insult, impairment of drainage, airway obstruction, and/or a defect in host defence. This results in damage to the muscular and elastic components of the bronchial wall both from the inciting infectious agent and from the host response. The latter may be mediated in part by inflammatory cytokines, nitric oxide, and neutrophilic proteases. Additionally, peribronchial alveolar tissue may be damaged, resulting in diffuse peribronchial fibrosis.

The result is abnormal bronchial dilatation with bronchial wall destruction and transmural inflammation. The most important functional finding of altered airway anatomy is severely impaired clearance of secretions from the bronchial tree.

### Tuberculosis

Tuberculosis (TB) has recently re-emerged as a public health problem. Most persons that are infected with *Mycobacterium tuberculosis* harbour the bacterium without symptoms but many develop active TB disease. Each year, 8 million people worldwide develop active TB and 3 million die.

Cases of TB dropped rapidly in the 1940s and 1950s when the first effective antibiotic therapies for TB were introduced. In 1985 the number of active TB cases in the United States began to rise again. Several forces, often interrelated, were behind. TB's resurgence. For example, individuals with HIV/AIDS are particularly vulnerable active TB.

The problem of multi drug resistance of *M. tuberculosis* is another factor in the re-emergence of the disease. Multidrug-resistant TB (MDR-TB) is much more difficult to cure. Treatment for MDR-TB often requires the use of special TB drugs, all of which can produce serious side effects. To cure MDR-TB, patients may have to take several antibiotics, at least three to which the bacteria still respond, every day for up to two years. However, even with this treatment, about 40% of MDR-TB will die, which is the same as for patients with standard TB who do not receive treatment. Palliative therapy for these patients is needed.

### Preparation of API

According to one aspect of the present invention a purified stable composition of API is provided. Preferably, a liquid composition of purified, stable of API is provided. International application WO 2005/027821, to one of the applicants of the present invention, provides pharmaceutical compositions comprising a purified, stable, active API in a form of a ready to use sterile solution. WO 2005/027821 also provides process, which combines removal of contaminating substances (i.e., lipids, lipoproteins and other proteins) and separation of active from inactive API by sequential chromatography steps. The process disclosed in that invention is suitable for a large-scale production of API.

The mixture of proteins from which the API is purified is preferably Cohn Fraction IV-1 paste, but can include other Cohn Fractions, separately or in combination; human blood plasma; plasma fractions; or any protein preparation containing API. For instance, the process is applicable to purification of recombinant human API from the milk of transgenic animals.

In that application, the mixture of proteins comprising API is dispersed in an aqueous medium, preferably water, at a ratio of between about 20 to about 35 liter per about 1 kg of source material, preferably Cohn Fraction IV-1 paste. The pH of the dispersion is adjusted to a pH range of from about 8.0 to about 9.5. The pH adjustment stabilizes the API and promotes the dissolution of the API in the dispersion, thereby increasing the production yield. Dispersion may take place at an elevated temperature of between 30°C and 40°C, for further increase in API solubility.

A particular advantage of that process is the elimination of contaminants or byproducts that otherwise compromise the efficiency of API purification processes. In particular, Cohn Fraction IV-1 paste preparations contain a significant amount of the lipoprotein Apo A-1, which has the effect of compromising column flow and capacity during purification. Other non-desired proteins such as albumin and transferrin are also present in the paste preparation. Removing a portion of such contaminants according to the invention disclosed in WO 2005/027821 is performed by two steps: (a) removing contaminating lipids and lipoproteins by lipid removal agent and (b) precipitating a portion of contaminating protein from the API-containing aqueous dispersion. The removal of contaminating proteins, without loss of API, enables a significant reduction in equipment scale, e.g., column size.

The precipitate that forms can be separated by conventional means such as centrifugation or filtration, and is then discarded. The supernatant is ready for further purification, for example an anion exchange resin. The API is then eluted from the column. The solution is treated to reduce its water content and change the ionic composition by conventional means such as by diafiltration, ultrafiltration, lyophilization, etc., or combinations thereof.

According to one embodiment, the API-containing effluent obtained after the first anion exchange chromatography is concentrated by ultrafiltration. The retentate is then diafiltered against pure water to reach conductivity within the range of from about 3.5 to about 4.5 mS/cm.

To further purify the API-containing solution obtained after the first anion exchange chromatography the solution is loaded on a cation exchange resin with the same type of buffer used for the anion-exchange step, having appropriate pH and conductivity such to allow the API to pass and be washed off with the buffer flow through, while contaminating substances are retained on the cation exchange resin.

The API-containing solution obtained after the cation exchange chromatography can be treated to reduce its water content. According to one embodiment, the solution is concentrated by ultrafiltration.

The ion-exchange chromatography is also used to separate active API from inactive API. That invention further comprises methods for separating active API from other contaminating substances, including solvent/detergent compounds used for viral inactivation. Such separation is achieved by the second anion exchange chromatography. The API eluted from the second anion exchange chromatography step is therefore not only highly active, but also highly pure.

Throughout the process of that invention only one type of buffer is used, with adjustment of pH and conductivity as required throughout the various process steps. The terms "one type" "same type" or "single type" of buffer are used interchangeably, and refer to a buffer with one specific anion species.

According to one embodiment, the buffer is any suitable acid/salt combination that provides acceptable buffer capacity in ranges of pH required throughout the API purification process. Preferably, the buffer used throughout the process is not citrate-based buffer, more preferably the buffer anion is acetate.

According to one embodiment, the process of that invention further comprises viral removal and/or viral inactivation steps. Methods for viral removal and inactivation are known in the art.

One method for viral removal is filtration, preferably nanofiltration, removing both enveloped and non-enveloped viruses. According to one embodiment, the viral removal step comprises filtration. According to another embodiment, the virus removal step is performed after the cation exchange chromatography. Typically, the cation exchange flow-through solution containing API is concentrated, and then nanofiltered.

According to one embodiment, the method of viral inactivation employed comprises a solvent/detergent (S/D) treatment. The viral inactivation step is preferably performed prior to loading the solution on the second anion exchange resin. According to one embodiment, the detergent used is polysorbate and the solvent is Tri-n-Butyl-Phosphate (TnBP). According to another embodiment, the polysorbate is polysorbate 80. According to one embodiment Polysorbate 80 may be added at from about 0.8% to about 1.3% volume per weight (v/w) of the resulting mixture and TnBP may be added from about 0.2% to about 0.4% weight per weight of the resulting mixture.

The solution containing active, purified API obtained after the second anion exchange chromatography can be further processed to obtain a pharmaceutical composition for therapeutic, diagnostic, or other uses. To prepare the product for therapeutic administration the process further comprises the steps of changing the ionic composition of the solution containing purified, active API to contain a physiologically compatible ion and sterilizing the resulted solution. The purified API obtained by the process of that invention is highly stable.

The pharmaceutical composition disclosed in WO 2005/0278 and used with the system and methods of the present invention comprises at least 90% pure, preferably 95% pure, more preferably 99% pure API. At least 90% of the API is in its active form.

### Pharmaceutical Compositions and Methods of Treatment

The term "pharmaceutical composition" is intended to be used herein in its broader sense to include preparations containing a protein composition in accordance with this invention used for therapeutic purposes. The pharmaceutical composition intended for therapeutic use should contain a therapeutic amount of API, i.e., that amount necessary for preventative or curative health measures.

As used herein, the term "therapeutically effective amount" refers to an amount of a protein or protein formulation or composition which is effective to treat a condition in a living organism to whom it is administered over some period of time.

Pharmaceutical compositions of the present invention may be manufactured by processes well known in the art, e.g. by means of conventional mixing, dissolving, granulating, grinding, pulverizing, dragee-making, levigating, emulsifying, encapsulating, entrapping or lyophilizing processes.

Pharmaceutical compositions for use in accordance with the present invention thus may be formulated in conventional manner using one or more acceptable diluents or carriers comprising excipients and auxiliaries, which facilitate processing of the active compounds into preparations, which can be used pharmaceutically. Proper formulation is dependent on the route of administration chosen. According to certain currently preferred embodiments, the pharmaceutical compositions of the present invention are formulated in a form suitable for inhalation.

The API-containing pharmaceutical compositions disclosed in WO 2005/027821 to one of the Applicants of the present invention is advantageous over hitherto known API-containing preparations, as the API is highly stable also when the composition is kept in a liquid from. Therefore, it is not necessary to lyophilize the API- preparation for stable storage in a form of a powder. Subsequently, there is no need to reinstate the powder to a liquid before use for parenteral administration or for inhalation. According to certain preferred embodiment, ready-to use liquid pharmaceutical composition is used with the system of the present invention. The liquid pharmaceutical composition can be packed in pre-sterilised unit dose vials containing 0.25 - 10 ml made of glass or polymeric materials or filled into polyethylen or any other suitable polymer vials manufactured for instance by a blow fill seal process, commonly used for ready to use inhalation solutions, having typical volume contents from 0.25 ml to 5 ml.

As described herein above, API is used for the treatment of pulmonary diseases. When administered intravenously, most of the API never reaches the lung. It has been estimated that only 2% of the intravenously administered dose reaches the lung (Hubbard & Crystal, 1990).

Therefore, administration of API by the inhalation route may be more beneficial as it reaches directly the lower respiratory tract. The present invention now discloses inhalation route which requires lower therapeutic doses of API and thus the scarce supply of human plasma-derived API would be available for the treatment of more patients. This route of administration may be also more effective in neutralizing neutrophil elastase, and thus the system and methods of the present invention are highly suitable for treating pulmonary diseases at periods of exacerbation. In addition, administration by inhalation is simpler and less stressful for the patient than the intravenous route and would reduce the burden on the local health care system (by requiring less clinical input).

Formulations of pharmaceutical compositions for administration by the route of inhalation are known in the art. In general, for administration by inhalation, the active ingredients are delivered in the form of an aerosol spray from a pressurized metered dose inhaler with the use of a suitable propellant, e.g., dichlorodifluoromethane, trichlorofluoromethane, dichloro-tetrafluoroethane or carbon dioxide, in powder form administered using a dry powder inhaler or, in aqueous liquid aerosol form using a nebulizer. Nebulizers for liquid aerosol delivery may be categorized as jet nebulizers operated by a pressurized flow of air using a portable compressor or central air supply in a hospital, ultrasonic nebulizers incorporating a piezo-crystal to provide the energy for generating the aerosol out of an ultrasonic fountain, and novel electronic nebulizers based on the principle of a perforated vibrating membrane, such as the eFlow^{®} nebulizer. All nebulization principles involve the aqueous solution being exposed to shear stresses, which may negatively affect the delicate nature of proteins such as API. However, the present invention now discloses that the vibrating membrane principle incorporated in the eFlow^{®} nebulizer can retain the integrity of the API protein structure and, thus, is best suited for pulmonary administration of the API.

Surprisingly, the present invention now discloses that using eFlow^{®} nebulizer for aerosolising the highly purified, stable API formulation (80 mg/4ml), about 70% of the loaded nominal dose can be delivered ex mouthpiece based on a simulated sinusoidal (inhalation: exhalation = 1: 1) breathing pattern applying 15 breaths/min and a tidal volume of 500 ml. About 60% of the nebulized dose is dissolved in droplets of 5 µm or less indicating that these droplets can be deposited in the lungs. The overall efficiency of the system of the present invention is superior over hitherto known inhalation systems, for example, the "Akita" inhalation management system associated with a customised PARI LC STAR nebulizer. Contrary to current proposed systems, the drug residue is only about 15% or less, whereas in a jet nebulizer like the LC STAR used in combination with Akita, the residue is at least 40% or more of the loaded dose resulting in a lower delivered dose in droplets of 5 µm or less compared to the system of the present invention. This novel feature allows for a more efficient and economic drug administration when using eFlow^{®} in combination with the API.

The operating conditions for delivery of a suitable inhalation dose vary according to the type of mechanical device employed. For some aerosol delivery systems, such as nebulizers, the frequency of administration and operating period will be dictated chiefly by the amount of the active composition (API according to the present invention) per unit volume in the aerosol. Typically, the higher the concentration of the protein in the nebulizer solution the shorter is the operating period. Some devices such as metered dose inhalers may produce higher aerosol concentrations than others and thus will be operated for shorter periods to give the desired result. The present invention now discloses that providing composition comprising less than about 10%, preferably less than about 5% of highly purified, stable API, nebulized in the eFlow^{®} nebulizer is highly efficient and effective in treating pulmonary disease.

According to one embodiment, the system and/or method of the present invention is used for treating pulmonary emphysema. It is known that patients with API deficiency have a low level of API and a high burden of neutrophils in their lower respiratory tracts. This observation supports the hypothesis that a deficiency of API predisposes a patient to emphysema by altering the balance between neutrophil elastase and anti-neutrophil elastase in the lower respiratory tract. Whereas normal persons have an adequate anti-neutrophil elastase screen to protect the lower respiratory tract, those with API deficiency do not, permitting the neutrophil elastase to destroy lung tissue. Thus, providing patient with endogenous API- deficiency with exogenic API at the correct dose and location can overcome the deleterious effects of such deficiency.

The principles of the invention may be better understood with reference to the nonlimiting examples below.

### EXAMPLES

Abbreviations: The following abbreviations are used herein.
- ACI:: Andersen Cascade Impactor.
- Aerosol Loss:: Amount of drug found on expiratory filters in a breath simulation experiment (mg or %), i.e. drug losses to the environment.

- API:: Alpha-1 Protease Inhibitor.
- Drug Residue:: Drug mass (mg) retained in the nebulizer after nebulization.
- GSD:: Geometric Standard Deviation, measure of the droplet size distribution.
- MMD:: Mass Median Diameter, calculated from the droplet size distribution determined by laser diffraction measurements (µm).
- MMAD:: Mass Median Aerodynamic Diameter, calculated from the droplet size distribution determined by cascade impaction measurements (µm).
- NGI:: Next Generation Pharmaceutical Impactor.
- RF:: Respirable Fraction, mass percentage of aerosol with droplet sizes ≤ 5µm.
- r.h.:: Relative humidity.

### Example 1: API uptake of API solution nebulized with eFlow® nebulizer

### Introduction

The study described hereinbelow examined the nebulization characteristics of an Alpha-1 Protease Inhibitor (API) solution when nebulized with eFlow^{®} electronic nebulizer (PARI GmbH, Germany, a company specialized in nasal and pulmonary drug delivery).

The set of experiments was carried out using a liquid formulation of Alpha-1 Proteinase Inhibitor (API), also suitable for injection (Kamada Ltd., Israel). The eFlow^{®} electronic inhaler used is an electronic nebulizer system based on a vibrating membrane principle, as disclosed in International Patent Application WO 01/34232. eFlow^{®} is designed to improve the delivery efficiency and utilization of an aerosolized drug. This is achieved by a vibrating membrane technology generating an aerosol with a very narrow droplet size distribution, expressed by a smaller GSD compared to jet nebulizers (1.7 vs. 2.1) (Balcke, et al., 2004). The unique configuration of the eFlow^{®} nebulizer results in a reduction in the percentage of coarse droplet and elevation of the percentage of very fine droplets, and thus facilitates a better targeting of the active pharmaceutical ingredient, particularly API, to the lung. This is particularly important for expensive drugs of limited availability, such as Alpha-1 Protease Inhibitor.

This study was designed to answer the following questions:
1. Is API protein activity retained upon nebulization via the eFlow^{®}?
2. What amount of API is carried in droplets smaller than 5 µm (*in-vitro* respirable dose)?
3. Can a desired droplet size of about 2.5 µm - 3.5 µm be achieved?
4. What amount of API reaches the mouth of a patient when providing an 80mg/4ml dose?
5. How much time is required for each nebulization session?

The delivery performance of nebulizers depends on the airflow pattern of a tidally breathing patient; therefore, a breath simulator was used to determine the dose of the drug delivered and the time required for nebulizung the entire amount of a drug composition, particularly a liquid drug composition. A standardized breathing manoeuvre was examined to determine the delivered dose, the drug residue in the device and the aerosol loss during exhalation (collected on "exhalation" filters to ensure recovery of the drug amount). An additional assay was conducted using a breathing pattern with reduced inhalation and a prolonged exhalation time, which mimics the breathing pattern of an emphysema patient.

### Materials

eFlow^{®} electronic nebulizers (PARI, Head No. 42008, 42012 and 42013)
Control Units (PARI, Ser. Nos. DBHSAB0043, DBH5AB0029 and DBH5AB0067)
API solution 2% (Kamada, Lot A and Lot B)
Filter pads (PARI, 041B0523) and filter casings with exhalation valve for expiratory filters (PARI)
Breath simulator (PARI, consisting of Controller Movtec 1A MSK, S/N 97442-2 and Sine Pump, S/N 230501 0500 with PARI COMPAS software v. 1.0)
Andersen Cascade Impactor (Copley Scientific, S/N 3429)
Next Generation Pharmaceutical Impactor (Copley Scientific, NGI-0169)
Software package for impactor analysis: CITDAS (Copley Scientific, Ver. 2.00)
Extraction buffer: The extraction buffer was prepared by dissolving 0.57g NaH₂PO₄-H₂O and 1.46g NaCl in water "Milli-Q" grade and adjusting the pH to 6.9 to 7.7. The solution volume was then completed with "Milli-Q" water to a final volume of 500 ml.

### Methods

### API content and activity

To examine the API drug content in formulations and test samples obtained from breath simulation and cascade impaction measurements, samples were collected on filter pads. Filter pads were extracted with phosphate buffer and the recovered API was analysed for total protein content, API activity and the content of API monomers. API activity was determined by measuring the extent of inhibition of proteolytic activity of porcine pancreatic elastase on the substrate succinyl-alanine-alanine-alanine-p-nitroanilide. The proteolytic activity was determined spectrophotometrically by the rate of appearance of the reaction product p-nitroaniline. The degree of inhibition of proteolytic activity induced by API is obtained by calculating the ratio of the reaction rate with non-inhibited porcine pancreatic elastase (no API in the reaction medium) to the reaction rate in the presence of API. The activity is expressed in mg API /ml. Presence of dimers/oligomers was examined by Size Exclusion HPLC, performed with Zorbax GF-250 GPC Column.

### Aerosol droplet size distribution

A. Assessment of the geometric droplet size distribution by laser diffraction (Malvern MasterSizerX): eFlow^{®} nebulizer was connected via a coupling chamber allowing entrainment of conditioned air through the nebulizer. Test conditions (T = 23 ± 1 °C, r.h. = 50 ± 5 %, flow rate = 20 L/min) were controlled and monitored. An average droplet size distribution was calculated from 5 single measuring intervals of 2000 sweeps every 10 seconds. Evaluation of laser diffraction data was performed according to the Mie-theory choosing the 2QAA presentation model supplied with the Malvern software.

B. The aerodynamic droplet size distribution was investigated by cascade impactor methods. Two different methods were used. The Andersen Cascade Impactor (ACI) is a traditional impactor type, which has been used in the past to characterize a wide range of different aerosols. The Next Generation Pharmaceutical Impactor (NGI) is a new impactor type, which has been specially designed for the characterization of pharmaceutical aerosols (Marple, et al., 2003a, 2003b, 2004) and has been recently introduced into the European Pharmacopoeia.

Two control samples (non-aerosolized API) were prepared by filling around 0.5 g of API solution into polypropylene (PP) tubes and adjusting the weight with extraction buffer to around 13 g. All samples were stored at 2°C to 8°C until analysis.

Samples were analyzed utilizing the API activity assay described above and evaluated with respect to droplet size distribution pattern, Mass Median Aerodynamic Diameter, Geometric Standard Deviation and Respirable Fraction (%<5µm) using the software package CITDAS.

### Determination of API delivered dose by breath simulation

A breath simulation apparatus was employed to characterize the drug delivery performance of the API/eFlow^{®} system under simulated breathing conditions. The nebulizer was loaded with a dose of 80mg/4ml and connected to the breath simulator, which generates the desired human breathing pattern.

The tests were performed according to the following protocol: The nebulizer was connected to a sinus pump (PARI breath simulator) mimicking a standard breathing pattern of an adult (500 ml tidal volume, 15 breaths/minute, inhalation:exhalation ratio 1:1, pattern S, Table 1). Alternatively, two modified patterns, which are used as models for impaired breathing patterns of emphysema patients, were employed (E1 and E2, Table 1).

Filters collecting the inhaled and exhaled aerosol ("inhalation" and "exhalation" filters, respectively) were installed between the nebulizer and the pump via a Y-piece. The nebulizer was filled with 4 ml of the API formulation for inhalation and run until all the solution was nebulized (nebulization time). Nebulization was interrupted at 3-minute intervals to change saturated filters. The aerosol (containing the API as the drug substance) was collected on the filters.

Measures of API deposited on the filters during the course of nebulization were used to calculate the API Delivered Dose, which is the amount of aerosol that would be inhaled by the patient ex mouthpiece, as well as the amount of aerosolized API lost during exhalation.

**Table 1: Breathing patterns used for breath simulation experiments**

| | S | E1 | E2 |
|---|---|---|---|
| Tidal volume (ml) | 500 | 450 | 425 |
| Inhalation (s) | 2.0 | 1.0 | 0.9 |
| Exhalation (s) | 2.0 | 2.5 | 3.0 |

Inhalation filters were changed after 3 and 6 minutes and after the end of nebulization. Proteins were extracted from the filters by placing the filters in a polypropylene (PP) test tube and adding 30 g of extraction buffer. The tubes were then shake at 250 rpm for 30 min., and the solution was separated from filters by decanting into new PP tubes. The amount of API remained as residue inside the nebulizer was measured by rinsing the nebulizer with 15g extraction buffer.

The value of the *in-vitro* respirable dose is based on the delivered dose taking into account the fraction of droplets which are in the respirable size range, i.e. in the range of <5µm. The *in-vitro* respirable dose was calculated by multiplying the Delivered Dose and the Respirable Fraction determined during aerosol droplet size characterization.

### Results and Discussion

### API activity following nebulization

Two different lots of API formulation were investigated. Table 2 summarizes the API characterizations before (control) ant after nebulization (test).

**Table 2: API characterization after nebulization of two different lots**

| | Lot A | | *Lot B* | |
|---|---|---|---|---|
| | Control | Test | Control | *Test* |
| Specific activity (%) | 0.97 | 0.87 | 1.09 | *1.03* |
| Monomer (%) | 94.3 | 96.4 | 98.3 | *98.0* |
| Dimer + Oligomer (%) | 5.6 | 3.6 | 1.5 | *1.8* |
| *Aggregates (%)* | *0.1* | *0.0* | *0.0* | *0.0* |

The results of the API activity tests show, that 90.0% of the API specific activity in lot A and 94.5% specific activity in lot B were retained. There was no increase in the amount of dimers and oligomers and no formation of aggregates.

### Breath simulation tests

Breath simulation experiments were performed with API using three eFlow^{®} electronic inhalers. Each device was tested in duplicate (n=6). A fill volume of 4.0 ml containing a nominal amount of 80 mg API was used. The breath simulation shows how the loaded dose is distributed during the inhalation, i.e. how much API is delivered to the patient at the mouthpiece, how much is lost during exhalation and how much remains in the nebulizer. The results of drug distribution are summarized in Table 3. The data shows mg of active API as determined by activity assay as described above. The table also includes the nebulization time as well as relative API amounts in percentage of the total loaded dose. The drug delivery profile shows a linear distribution over time and an average nebulization time of 9 ± 0.1 min (Figure 1).

**Table 3: API distribution data determined by breath simulation (n=6)**

| | **API (activity)** | |
|---|---|---|
| | mean | ±SD |
| Delivered Dose (mg) | 65.3 | 3.2 |
| Drug residue (mg) | 14.4 | 3.6 |
| Aerosol loss (mg) | 13.9 | 2.5 |
| Nebulization time (min) | 9.0 | 1.0 |
| Delivered Dose (%) | 70.0 | 3.2 |
| Drug residue (%) | 15.4 | 3.9 |
| Aerosol loss (%) | 14.9 | 2.6 |

To test whether the Delivered Dose is affected by the breathing pattern, additional experiments were performed using breathing patterns that are thought to represent a patient whose breathing is impaired by emphysema, E1 and E2 patterns (see table 1). These tests were done in duplicate for each pattern. The results are summarized in Table 4, wherein pattern E1 was used for run 1 and 2 and pattern E2 for run 3 and 4.

**Table 4: API distribution data after nebulization with emphysema breathing pattern**

| | API (activity) | | | | | |
|---|---|---|---|---|---|---|
| | Run 1 | Run 2 | Run 3 | Run 4 | Mean | ±SD |
| Delivered Dose (mg) | 48.2 | 46.5 | 47.1 | 47.0 | 47.2 | 0.6 |
| Drug residue (mg) | 13.2 | 12.7 | 15.2 | 16.9 | 14.5 | 1.7 |
| Aerosol loss (mg) | 16.2 | 17.2 | 12.1 | 0.0* | 11.4 | 6.8 |
| Nebulization time (min) | 10.1 | 10.2 | 7.9 | 7.9 | 9.0 | 1.1 |
| Delivered Dose (%) | 57.1 | 55.2 | 55.9 | 55.5 | 55.9 | 0.7 |
| Drug residue (%) | 15.6 | 15.1 | 18.1 | 19.9 | 17.2 | 1.9 |
| Aerosol loss (%) | 19.1 | 20.4 | 14.3 | 0.0¹ | 13.5 | 8.1 |

| | | | | | | |
|---|---|---|---|---|---|---|
| * No API was detected on the filter: considered as due to an error during filter extraction. | | | | | | |

The additional experiments show delivered dose values in the range from 55% to 57% of loaded dose. There appears to be no difference in delivered dose values between the two patterns. Hence, mean values were calculated from all four runs. The series of breath simulations show that around 70% of the loaded dose can be delivered exmouthpiece of the nebulizer with a breathing pattern reflecting a normally breathing patient and around 56% can still be delivered for a severely impaired breathing pattern.

### Droplet size distribution

The droplet size distribution profiles for API nebulized by the eFlow^{®} was assessed by two different cascade impaction methods and by laser diffractometry as described herein: All impaction tests were performed with 4 ml API solution (20 mg/ml) nebulized by three eFlow^{®} devices, each in duplicate (n=6).

### Andersen cascade impaction (ACI)

Aerodynamic droplet size distribution was analyzed using an Andersen Cascade Impactor (ACI) fitted with an USP-throat (Copley Scientific Instruments, Nottingham, UK). The ACI was equilibrated at 18°C using a water bath. Environmental conditions were controlled at 23°C and 50% relative humidity. The nebulizer was filled with 4 ml of formulation and connected to the USP-throat. The airflow rate through the nebulizer and impactor was set at 28.3 L/min. The nebulizer was operated until nebulization was complete. Then the ACI was disassembled, plates and USP-throat were rinsed with 10 ml of phosphate buffer. The drug content was analyzed for each impactor stage and the USP throat separately. The Mass Median Aerodynamic Diameter (MMAD) was calculated according to USP methods, using the CITDAS software package (Copley Scientific Instruments, Nottingham, UK).

The impactor was operated at its calibration flow rate of 28.3 L/min at 22°C ±2°C and at 50% ± 5% relative humidity (r.h.) according to a published test set up (Keller et al., 2002; Jaurnig, et al., 2004). Proteins from the ACI plates were extracted with 10 ml extraction buffer for 30 min. The ACI Inlet was rinsed with 10 g and the nebulizer with 15 g of extraction buffer (weight recorded). Droplet size distribution upon nebulization of 80mg/4ml API by three eFlow^{®} devices (each in a duplicate, n=6) are summarised in table 5 below.

**Table 5: Droplet size distribution upon Nebulization of 80 mg/ 4ml API with ACI**

| ACI 28.3L/min | Deposition [mg] | | | | | | Mean |
|---|---|---|---|---|---|---|---|
| 22°C, 50% r.h. | | | | | | | |
| | run 1 | run 2 | run 3 | run 4 | run 5 | run 6 | [mg] |
| ACI induction port (artificial throat) | 0.1 | 0 | 0 | 0 | 0 | 0 | 0.0 |
| Stage 0 (< 10.0 µm) | 0.7 | 0.8 | 0.5 | 0.5 | 0.8 | 0.4 | 0.6 |
| Stage 1 (< 9.0 µm) | 2.9 | 2.6 | 1.4 | 2.4 | 3.1 | 1.5 | 2.3 |
| Stage 2 (< 5.80 µm) | 5.3 | 5.7 | 4.5 | 6.8 | 6.2 | 4.6 | 5.5 |
| Stage 3 (< 4.70 µm) | 23.6 | 27 | 25.7 | 25 | 22.4 | 25.4 | 24.9 |
| Stage 4 (< 3.30 µm) | 22.4 | 25.4 | 31.8 | 23.5 | 21.9 | 31.6 | 26.1 |
| Stage 5 (< 2.10 µm) | 4.6 | 7.2 | 7 | 4.1 | 8.2 | 6.7 | 6.3 |
| Stage 6 (< 1.10 µm) | 0.3 | 0.4 | 0.3 | 0.2 | 0.5 | 0.4 | 0.4 |
| Stage 7 (< 0.65 µm) | 0.1 | 0.1 | 0.1 | 0 | 0.1 | 0 | 0.1 |
| Filter (< 0.43 µm) | 0 | 0 | 0 | 0 | 0 | 0 | 0.0 |

It is apparent from the ACI data in the table above, that the ballistic momentum of the aerosol is negligible, since no drug is lost in the ACI-induction port which is acting as an artificial throat. The majority of drug (about 57.7 mg or 72.1%) of the nominal dose is contained in droplets of about 1.1 - 4.7 µm allowing for a deep lung deposition of API into the lung periphery, which is the target site for API activity for providing an optimal therapeutic effect.

### Next Generation Impactor (NGI)

Aerodynamic droplet size distribution was also analyzed using a Next Generation Pharmaceutical Impactor (NGI) fitted with a USP-throat (Copley Scientific Instruments, Nottingham, UK). eFlow^{®} was filled with 4 ml of API formulation and connected to the USP-throat. The nebulizer was operated until nebulization was completed, and then the NGI was disassembled. The API content was analyzed for each impactor stage and the USP throat separately. The Mass Median Aerodynamic Diameter (MMAD) was calculated according to USP methods, using the software package CITDAS (Copley Scientific Instruments, Nottingham, UK).

The NGI was operated at a flow of 15 ± 0.5 1/min at 22°C ±2°C and at 50% ±5% relative humidity (r.h.). The NGI cups were weighted before and after loading. The API on the single stages was extracted with 10 ml extraction buffer for 30 min. The NGI inlet was rinsed with 20 g and the nebulizer with 15 g extraction buffer (weight recorded). Droplet size distribution upon nebulization of 80 mg/ 4ml API by three Flow devices (each in a duplicate, n=6) are summarised in table 6 below.

**Table 6: Nebulization of 80 mg/ 4ml API with NGI**

| NGI 15L/min | Deposition [mg] | | | | | | Mean |
|---|---|---|---|---|---|---|---|
| 22°C, 50% r.h. | | | | | | | |
| | run 1 | run 2 | run 3 | run 4 | run 5 | run 6 | [mg] |
| Induction Port | 0.2 | 0.2 | 0.2 | 0.1 | 0.2 | 0.2 | 0.2 |
| Stage 1 (< 14.1 µm) | 1.2 | 1 | 1 | 1.1 | 1.1 | 0.9 | 1.1 |
| Stage 2 (<8.61 µm) | 1 | 1 | 8 | 1.1 | 0.8 | 0.8 | 2.1 |
| Stage 3 (< 5.36 µm) | 5.4 | 5.2 | 3.5 | 6 | 4.2 | 3.4 | 4.6 |
| Stage 4 (< 3.30 µm) | 24.4 | 21.7 | 20.5 | 24.5 | 23.4 | 19.4 | 22.3 |
| Stage 5 (< 2.08 µm) | 31.3 | 27.5 | 34.6 | 28.5 | 34.7 | 37.1 | 32.3 |
| Stage 6 (< 1.36 µm) | 5.9 | 5.8 | 7.6 | 8.2 | 7.7 | 8.8 | 7.3 |
| Stage 7 (< 0.98 µm) | 0.2 | 0.6 | 0.7 | 0.8 | 0.9 | 0.8 | 0.7 |
| MOC (< 0.37 µm) | 0.2 | 0.5 | 0.5 | 0.6 | 0.5 | 0.7 | 0.5 |

It is apparent from the NGI data, that the ballistic momentum of the aerosol is negligible, since only 0.2 mg (i.e. 0.25%) of the nominal dose is lost and captured in the NGI-induction port which is acting as an artificial throat. The majority of drug (about 61.9 mg or 77.4%) of the nominal dose is contained in droplets of about 1.36 - 3.3 µm. These results further support the results obtained with the cascade impactors, showing that administering API by inhalation using the eFlow^{®} nebulizer is highly effective in targeting the API to the lung alveoli, where its activity is mostly required.

### Laser Diffraction

The geometric droplet size distribution was characterized by a diffractometer Mastersizer X (Malvern Instruments, Herrenberg, Germany). Environmental conditions were controlled at 23°C and 50% relative humidity. The airflow rate through the nebulizer and diffractometer was set at 20 l/min. The Mass Median Diameter (MMD) was calculated according to the Mie-theory choosing the 2QAA presentation model supplied with the Malvern software.

A comparison of size distribution parameters after API nebulization derived by laser diffraction or cascade impaction with NGI and ACI impactors is shown in Table 7.

**Table 7: Droplet size distribution parameters after API nebulization derived by laser diffraction or cascade impaction with NGI and ACI impactors (n=6)**

| | Laser Diffraction | | NGI | | *ACI* | |
|---|---|---|---|---|---|---|
| | mean | SD | mean | SD | mean | *SD* |
| MMD/MMAD (µm) | 3.1 | 0.1 | 3.1 | 0.1 | 3.3 | *0.1* |
| GSD | 1.5 | 0.0 | 1.5 | 0.1 | 1.4 | *0.1* |
| *RF (<5µm) (%)* | *87.9* | *2.2* | *87.4* | *2.7* | *90.3* | *2.6* |

As is apparent from Table 7, the results obtained from laser diffraction, NGI and ACI are in a good agreement. Characterization of the aerosol droplet size distribution by three different methods shows a mass median diameter size in the range of about 3.1 µm to about 3.3 µm and a respirable fraction of droplets below 5 µm between 87% and 90% with the API solution and the eFlow^{®} electronic nebulizer. Multiplying these values with the delivered dose found in breath simulation measurements (see Table 4 hereinabove), the respirable fraction ranges between 57-59 mg per inhalation. Furthermore, remarkably low geometric standard deviations (GSD) have been found indicating a very narrow droplet size distribution allowing for targeted delivery of the drug to the deep lungs.

### Respirable Dose, Drug Delivery Rate and Respirable Drug Delivery Rate

The value of the in-vitro respirable dose is derived from the delivered dose determined in the breath simulation experiment and the respirable fraction, i.e. the proportion of the API drug disposed in droplets having a size of <5 µm. Assessment of respirable fraction by three different droplet-sizing methods gave very similar results (between 87.4 and 90.3 %) with an average of 88.5 ± 2.15 % across all methods. This value is used for the calculation of the respirable dose. The drug delivery rate and the respirable drug delivery rate are obtained by dividing the respective doses by the nebulization time. Table 9 summarizes the data for n=6.

**Table 9: Respirable Dose, Drug Delivery Rate and Respirable Drug Delivery Rate**

| | API | |
|---|---|---|
| | Mean | ±SD |
| Respirable Dose (mg <5µm) | 57.8 | 2.90 |
| Respirable Dose (% of charged amount <5µm) | 61.9 | 2.79 |
| Drug Delivery Rate (mg/min) | 7.4 | 0.98 |
| Respirable Drug Delivery Rate (mg <5µm /min) | 6.5 | 0.74 |

### Summary and Conclusion

The present *in-vitro* study investigated the nebulization of Alpha-1 Protease Inhibitor (API) solution in combination with the eFlow^{®}, a novel electronic nebulizer.

The drug protein was not significantly damaged by the nebulization process. API activity in the two tested lots after nebulization by eFlow^{®} was found to be 90.0% to 94.5% of the original activity.

The droplet size distribution obtained from the eFlow^{®} was investigated by cascade impaction and laser diffraction. Cascade impaction was conducted using the Next Generation Pharmaceutical Impactor and the Andersen Cascade Impactor. Laser Diffraction was conducted using a Malvern Mastersizer X. The target range of the droplet size was between 1 µm and 4 µm. Characterization by three different methods showed a median droplet size, MMD or MMAD, between 3.1 µm and 3.3 µm, meeting the desired target, and a respirable fraction of 87% to 90%. The GSD, which indicates the scattering of the droplet size distribution, was around 1.5, representing a very narrow size distribution.

*In-vitro* breath simulation tests were conducted to assess treatment time and API delivery, using a standardized breathing pattern representing an adult patient. The volume of API solution filled into the eFlow^{®} was 4.0 ml (equivalent to a nominal dose of 80 mg). The nebulization time was 9.0 ± 0.1 min. An amount of around 65 mg (70%) of active API can be delivered at the mouthpiece of the nebulizer. Combining this with the respirable fractions determined by droplet size distribution, an *in-vitro* respirable dose of around 58 mg (62%) can be calculated. Additional tests with a breathing pattern thought to represent a patient suffering from severe emphysema still gave an *in-vitro* delivered dose of 47 mg (56%).

The aforementioned *in-vitro* study has shown that nebulization of API by eFlow^{®} produces an aerosol suitable to reach the patients lungs. The inhalation of API nebulized with the eFlow^{®} delivers the drug molecules directly to the site of action and reduces the required amount of the scarce and expensive drug. Inhalation of API can help to offer an easy, quick and efficient therapy and can enable more patients suffering from pulmonary disease to receive treatment.

The foregoing description of the specific embodiments will so fully reveal the general nature of the invention that others can, by applying current knowledge, readily modify and/or adapt for various applications such specific embodiments without undue experimentation. Adaptations and modifications should and are intended to be comprehended within the meaning and range of the disclosed embodiments. It is to be understood that the phraseology or terminology employed herein is for the purpose of description and not of limitation. The means, materials, and steps for carrying out various disclosed systems, methods and functions may take a variety of alternative forms without departing from the invention.

### REFERENCES

Balcke, A., A. Bucholski, R. Waldner, M. Knoch, and M. Keller, Performance characteristics of colistimethate sodium solution (Colistin) nebulized by jet nebulizers compared to the novel electronic inhaler (eFlow®), 27th European Cystic Fibrosis Conference, Birmingham UK, June 13th - 17th, 2004.
Hubbard RC, Crystal RG. Strategies for aerosol therapy of alpha 1-antitrypsin deficiency by the aerosol route. Lung 168 Suppl:565-78, 1990
Jauernig, J., S. Ohl, M. Knoch, and M. Keller, Effects of test set-up, formulation and nebulizer type on aerodynamic droplet characteristics. Proceedings of the Respiratory Drug Delivery IX. Palm Desert CA, USA, 609-612, 2004.
Keller, M., J. Jauernig, F.-C. Lintz, and M. Knoch, Nebulizer Nanosuspensions: Important Device and Formulation Intractions. Proceedings of the Respiratory Drug Delivery VIII, Tuscon, USA, May 12th-16th, 197-206,2002.
Knoch, M., and M. Keller: The customised electronic nebulizer: a new category of liquid aerosol drug delivery systems. Expert Opinion in Drug Delivery 2(2):377-390 2005.
Marple, V. A., D.L. Roberts, F.J. Romay, N.C. Miller, K.G. Truman, M. Van Oort, B. Olsson, M.J. Holroyd, J.P. Mitchell and D. Hochrainer: Next Generation Pharmaceutical Impactor (A New Impactor for Pharmaceutical Inhaler Testing). Part I: Design. J. Aerosol Med. Vol. 16, No.3, 2003a, p. 283 - 299.
Marple, V. A., B.A. Olson, K. Santhanakrishnan, J.P. Mitchell, S.C. Murray and B.L. Hudson-Curtis: Next Generation Pharmaceutical Impactor (A New Impactor for Pharmaceutical Inhaler Testing). Part II: Archival Calibration. J. Aerosol Med. Vol. 16, No.3, 2003b, p. 301- 324.
Marple, V.A., B.A. Olson, K. Santhanakrishnan, D.L. Roberts, J.P. Mitchell and B.L. Hudson-Curtis: Next Generation Pharmaceutical Impactor: A New Impactor for Pharmaceutical Inhaler Testing. Part III. Extension of Archival Calibration to 15 L/min. Journal of Aerosol Medicine, 17, 4, 2004, p. 335-343.
McElvaney N.G., R.C. Hubbard, P. Birrer, M.S. Chernick, D.B. Caplan, M.M. Frank and R.G. Crystal. Aerosol alpha 1-antitrypsin treatment for cystic fibrosis. Lancet. 1991 337(8738):392-4.

## Claims

1. A system for the treatment of pulmonary diseases comprising:
a) a pharmaceutical composition comprising a purified, stable, active alpha-1 proteinase inhibitor (API) produced by the liver and secreted into the circulatory system in a ready to use sterile solution, wherein the purified, stable alpha-1 proteinase inhibitor is at least 90% pure; and
b) an inhalation nebulizer comprising
i) an aerosol generator comprising: a liquid storage container comprising the liquid pharmaceutical composition; a diaphragm having a first side and an opposite second side, the diaphragm having a plurality of openings extending therethrough from the first side to the second side, where the first side is connected to the liquid storage container such that the liquid filled into the liquid storage container comes into contact with the first side of the diaphragm; and a vibration generator capable of vibrating the diaphragm so that the liquid filled into the liquid storage container is atomized on the second side of the diaphragm through the openings of the diaphragm;
ii) a mixing chamber into which the aerosol generator expels said aerosol, the mixing chamber in contact with the second side of the diaphragm;
iii) an inhalation valve that is open to allow an inflow of ambient air into the mixing chamber during an inhalation phase and is closed to prevent escape of said aerosol from the mixing chamber during an exhalation phase; and
iv) an exhalation valve that is open to allow the discharge of the respiratory air of a patient into the surroundings during the exhalation phase and is closed to prevent the inflow of ambient air during the inhalation phase;
wherein the pharmaceutical composition is nebulized by the inhalation nebulizer to form an aerosol composition and wherein at least 90% of the pure, nebulized alpha-1 proteinase inhibitor is in its active form.

2. The system according to claim 1,
wherein the purified, stable alpha-1 proteinase inhibitor is purified from an unpurified mixture of proteins by a process comprising elution from a plurality of ion exchange resins, preferably wherein the purified, stable alpha-1 proteinase inhibitor is purified from an unpurified mixture of proteins by a process comprising elution from at least two anion exchange resins and at least one cation exchange resin; or
wherein the pH of the pharmaceutical composition is in the range of 6.5-7.5; or
wherein the concentration of the purified, stable alpha-1 proteinase inhibitor is less than 10% w/v, preferably wherein the concentration of the purified, stable alpha-1 proteinase inhibitor is between 1% to 5% ,more preferably wherein the concentration of the purified, stable alpha-1 proteinase inhibitor is about 2%.

3. The system according to claim 1,
wherein the purified, stable alpha-1 proteinase inhibitor is at least 95% pure; or
wherein the purified, stable alpha-1 proteinase inhibitor is at least 99% pure.

4. The system according to claim 1, wherein the mass median diameter of the aerosol droplets produced by the inhalation nebulizer is less than about 5 µm; or
wherein the mass median diameter of the aerosol droplets is between 1.0 µm and 4.0 µm; or
wherein the mass median diameter of the aerosol droplets is between 2.0 µm and 3.5 µm; or
wherein the mass median diameter of the aerosol droplets is between 2.5 µm and 3.3 µm.

5. The system according to claim 4, wherein the geometric standard deviation (GSD) is lower than about 2.0; or
wherein the geometric standard deviation (GSD) is lower than about 1.7.

6. The system according to claim 1, wherein at least 60% of API is dissolved in aerosol droplets of less than about 5 µm.

7. The system according to claim 1, wherein the pulmonary disease is selected from the group consisting of emphysema; chronic obstructive pulmonary disorder (COPD); bronchiectasis; tuberculosis; parenchymatic or fibrotic associated lung diseases and disorders including cystic fibrosis, interstitial pulmonary fibrosis and sarcoidosis; and pulmonary diseases secondary to HIV, preferably
wherein emphysema is inherited emphysema, ore preferably wherein the parenchymatic or fibrotic associated lung disease is cystic fibrosis.

8. Use of a pharmaceutical composition comprising a purified, stable, active alpha-1 proteinase inhibitor (API) produced by the liver and secreted into the circulatory system in a form of a ready to use sterile solution, wherein the stable alpha-1 proteinase inhibitor is at least 90% pure; and b) an inhalation nebulizer comprising
i) an aerosol generator comprising: a liquid storage container comprising the liquid pharmaceutical composition; a diaphragm having a first side and an opposite second side, the diaphragm having a plurality of openings extending therethrough from the first side to the second side, where the first side is connected to the liquid storage container such that the liquid filled into the liquid storage container comes into contact with the first side of the diaphragm; and a vibration generator capable of vibrating the diaphragm so that the liquid filled into the liquid storage container is atomized on the second side of the diaphragm through the openings of the diaphragm;
ii) a mixing chamber into which the aerosol generator expels said aerosol, the mixing chamber in contact with the second side of the diaphragm;
iii) an inhalation valve that is open to allow an inflow of ambient air into the mixing chamber during an inhalation phase and is closed to prevent escape of said aerosol from the mixing chamber during an exhalation phase; and
iv) an exhalation valve that is open to allow the discharge of the respiratory air of a patient into the surroundings during the exhalation phase and is closed to prevent the inflow of ambient air during the inhalation phase;
for the preparation of a system comprising said pharmaceutical composition and said nebulizer wherein the pharmaceutical composition is nebulized by the inhalation nebulizer to form an aerosol composition, for administering to a subject a therapeutically effective amount of the aerosol composition comprising alpha-1 proteinase inhibitor, wherein at least 90% of the pure, nebulized alpha-1 proteinase inhibitor is in its active form.

9. The use according to claim 8, wherein the system is for treating a pulmonary disease at a phase of acute exacerbation.

10. The use according to any one of claims 8-9, wherein the pulmonary disease is selected from the group consisting of emphysema; chronic obstructive pulmonary disorder (COPD); bronchiectasis; tuberculosis; parenchymatic or fibrotic associated lung diseases and disorders including cystic fibrosis, interstitial pulmonary fibrosis and sarcoidosis; and pulmonary diseases secondary to HIV.

11. The use according to claim 10,
wherein emphysema is inherited emphysema; or
wherein the parenchymatic or fibrotic associated lung disease is cystic fibrosis.

12. The use according to claim 11
wherein at least 50% of the API is delivered to the subject; or
wherein at least 60% of the API is delivered to the subject; or
wherein at least 70% of the API is delivered to the subject.

13. The use according to claim 12, wherein the API is absorbed by lung tissues of the subject.

14. The use according to claim 8, wherein the subject is a human subject.

## Patentansprüche

1. System für die Behandlung von Lungenerkrankungen, umfassend:
a) eine pharmazeutische Zusammensetzung umfassend einen gereinigten, stabilen, aktiven Alpha-1 Proteinaseinhibitor (API), der von der Leber erzeugt und in das Kreislaufsystem sekretiert wird, in einer gebrauchsfertigen Sterillösung, worin der gereinigte, stabile Alpha-1 Proteinaseinhibitor mindestens 90% rein ist; und
b) einen Inhalationszerstäuber, umfassend
i) einen Aerosolgenerator, umfassend: einen Aufbewahrungsbehälter für eine Flüssigkeit, der die flüssige pharmazeutische Zusammensetzung umfasst; eine Membran mit einer ersten Seite und einer abgewandten zweiten Seite, worin die Membran mehrere Öffnungen aufweist, die von der ersten Seite zu der zweiten Seite dadurch verlaufen, worin die erste Seite mit dem Aufbewahrungsbehälter für eine Flüssigkeit verbunden ist, so dass die in den Aufbewahrungsbehälter für eine Flüssigkeit gefüllte Flüssigkeit mit der ersten Seite der Membran in Kontakt gerät; und ein Vibrationsgenerator, der die Membran in Schwingung versetzen kann, so dass die in den Aufbewahrungsbehälter für eine Flüssigkeit gefüllte Flüssigkeit durch die Öffnungen der Membran auf der zweiten Seite der Membran zerstäubt ist;
ii) eine Mischkammer, in die der Aerosolgenerator das Aerosol ausstößt, worin die Mischkammer mit der zweiten Seite der Membran in Kontakt steht;
iii) ein Inhalationsventil, das geöffnet ist, um ein Einströmen von Umgebungsluft in die Mischkammer während einer Inhalationsphase zu ermöglichen, und geschlossen ist, um ein Entweichen des Aerosols aus der Mischkammer während einer Ausatmungsphase zu verhindern; und
iv) ein Exhalationsventil, das geöffnet ist, um den Austritt der Atemluft eines Patienten in die Umgebung während der Ausatmungsphase zu ermöglichen, und geschlossen ist, um das Einströmen von Umgebungsluft während der Inhalationsphase zu verhindern;
worin die pharmazeutische Zusammensetzung durch den Inhalationszerstäuber zerstäubt wird, um eine Aerosolzusammensetzung zu bilden, und worin mindestens 90% des reinen, zerstäubten Alpha-1 Proteinaseinhibitors in seiner aktiven Form vorliegt.

2. System nach Anspruch 1,
worin der gereinigte, stabile Alpha-1 Proteinaseinhibitor aus einem ungereinigten Proteingemisch durch ein Verfahren gereinigt wird, das Elution von mehreren Ionenaustauschharzen umfasst, worin vorzugsweise der gereinigte, stabile Alpha-1 Proteinaseinhibitor von einem ungereinigten Proteingemisch durch ein Verfahren gereinigt wird, das Elution von mindestens zwei Anionenaustauschharzen und mindestens einem Kationenaustauschharz umfasst; oder
worin der pH der pharmazeutischen Zusammensetzung in dem Bereich von 6,5-7,5 liegt; oder
worin die Konzentration des gereinigten, stabilen Alpha-1 Proteinaseinhibitors weniger als 10% Masse/Volumen beträgt, worin die Konzentration des gereinigten, stabilen Alpha-1 Proteinaseinhibitors vorzugsweise zwischen 1% bis 5% beträgt, worin die Konzentration des gereinigten, stabilen Alpha-1 Proteinaseinhibitors bevorzugter ungefähr 2% beträgt.

3. System nach Anspruch 1,
worin der gereinigte, stabile Alpha-1 Proteinaseinhibitor mindestens 95% rein ist; oder
worin der gereinigte, stabile Alpha-1 Proteinaseinhibitor mindestens 99% rein ist.

4. System nach Anspruch 1, worin der mittlere Massendurchmesser der durch den Inhalationszerstäuber erzeugten Aerosoltröpfchen weniger als ungefähr 5 µm beträgt; oder
worin der mittlere Massendurchmesser der Aerosoltröpfchen zwischen 1,0 µm und 4,0 µm beträgt; oder
worin der mittlere Massendurchmesser der Aerosoltröpfchen zwischen 2,0 µm und 3,5 µm beträgt; oder
worin der mittlere Massendurchmesser der Aerosoltröpfchen zwischen 2,5 µm und 3,3 µm beträgt.

5. System nach Anspruch 4, worin die geometrische Standartabweichung (GSD) geringer als ungefähr 2,0 ist; oder
worin die geometrische Standartabweichung (GSD) geringer als ungefähr 1,7 ist.

6. System nach Anspruch 1, worin mindestens 60% des API in Aerosoltröpfchen von weniger als ungefähr 5 µm gelöst ist.

7. System nach Anspruch 1, worin die Lungenerkrankung ausgewählt ist aus der Gruppe bestehend aus Emphysem; chronisch obstruktiver Lungenerkrankung (COPD); Bronchiektasie; Tuberkulose; parenchymatische oder mit Fibrose zusammenhängende Lungenerkrankungen und Erkrankungen, die zystische Fibrose, interstitielle Lungenfibrose und Sarcoidose umfassen; und Lungenerkrankung in Folge von HIV,
worin das Emphysem vorzugsweise vererbtes Emphysem ist, oder worin die parenchymatische oder mit Fibrose zusammenhänge Lungenerkrankung vorzugsweise zystische Fibrose ist.

8. Verwendung (a) einer pharmazeutischen Zusammensetzung umfassend einen gereinigten, stabilen, aktiven Alpha-1 Proteinaseinhibitor (API), der von der Leber erzeugt wird und in das Kreislaufsystem sekretiert wird, in einer Form einer gebrauchsfertigen Sterillösung, worin der stabile Alpha-1 Proteinaseinhibitor mindestens 90% rein ist; und b) eines Inhalationszerstäubers, umfassend
i) einen Aerosolgenerator umfassend: einen Aufbewahrungsbehälter für eine Flüssigkeit, der die flüssige pharmazeutische Zusammensetzung umfasst; eine Membran mit einer ersten Seite und einer abgewandten zweiten Seite, worin die Membran mehrere Öffnungen aufweist, die von der ersten Seite zu der zweiten Seite dadurch verlaufen, worin die erste Seite mit dem Aufbewahrungsbehälter für eine Flüssigkeit verbunden ist, so dass die in den Aufbewahrungsbehälter für eine Flüssigkeit gefüllte Flüssigkeit mit der ersten Seite der Membran in Kontakt gerät; und einen Vibrationsgenerator, der die Membran in Schwingung versetzen kann, so dass die in den Aufbewahrungsbehälter für eine Flüssigkeit gefüllte Flüssigkeit durch die Öffnungen der Membran auf der zweite Seite der Membran zerstäubt ist;
ii) eine Mischkammer, in die der Aerosolgenerator das Aerosol ausstößt, worin die Mischkammer mit der zweite Seite der Membran in Kontakt steht;
iii) ein Inhalationsventil, das geöffnet ist, um ein Einströmen von Umgebungsluft in die Mischkammer während einer Inhalationsphase zu ermöglichen, und geschlossen ist, um Entweichen des Aerosols aus der Mischkammer während einer Ausatmungsphase zu verhindern; und
iv) ein Exhalationsventil, das geöffnet ist, um den Austritt der Atemluft eines Patienten in die Umgebung während der Ausatmungsphase zu ermöglichen, und geschlossen ist, um das Einströmen von Umgebungsluft während der Inhalationsphase zu verhindern;
für die Herstellung eines Systems, das die pharmazeutische Zusammensetzung und den Zerstäuber umfasst, worin die pharmazeutische Zusammensetzung durch den Inhalationszerstäuber zerstäubt wird, um eine Aerosolzusammensetzung zu bilden, um einem Patienten eine therapeutisch wirksamen Menge der Alpha-1 Proteinaseinhibitor umfassenden Aerosolzusammensetzung zu verabreichen, worin mindestens 90% des reinen, zerstäubten Alpha-1 Proteinaseinhibitors in seiner aktiven Form ist.

9. Verwendung nach Anspruch 8, worin das System für die Behandlung einer Lungenerkrankung in einer Phase einer akuten Exazerbation ist.

10. Verwendung nach einem der Ansprüche 8-9, worin die Lungenerkrankung ausgewählt ist aus der Gruppe bestehend aus Emphysem; chronisch obstruktiver Lungenerkrankung (COPD); Bronchiektasie; Tuberkulose; parenchymatische oder mit Fibrose zusammenhängender Lungenerkrankungen und Erkrankungen, die zystische Fibrose, interstitielle Lungenfibrose und Sarcoidose umfassen; und Lungenerkrankung in Folge von HIV.

11. Verwendung nach Anspruch 10,
worin das Emphysem ein vererbtes Emphysem ist; oder
worin die parenchymatische oder mit Fibrose zusammenhänge Lungenerkrankung zystische Fibrose ist.

12. Verwendung nach Anspruch 11,
worin mindestens 50% des API an den Patienten verabreicht werden; oder
worin mindestens 60% des API an den Patienten verabreicht werden; oder
worin mindestens 70% des API an den Patienten verabreicht werden.

13. Verwendung nach Anspruch 12, worin der API durch Lungengewebe des Patienten absorbiert wird.

14. Verwendung nach Anspruch 8, worin der Patient ein menschlicher Patient ist.

## Revendications

1. Système destiné au traitement de maladies pulmonaires, qui comprend :
a) une composition pharmaceutique comprenant un inhibiteur de la protéinase alpha-1 (API) purifié, stable et actif produit par le foie et sécrété dans le système circulatoire sous forme de solution stérile prête à l'emploi, dans laquelle l'inhibiteur de la protéinase alpha-1 purifié et stable présente une pureté d'au moins 90 % ; et
b) un nébuliseur d'inhalation qui comprend :
i) un générateur d'aérosol qui comprend : un récipient de stockage de liquide qui comprend la composition pharmaceutique liquide ; un diaphragme ayant un premier côté et un second côté opposé ; le diaphragme ayant une pluralité d'ouvertures s'étendant à travers celui-ci, du premier côté au second côté, le premier côté étant connecté au récipient de stockage de liquide de telle sorte que le liquide rempli dans le récipient de stockage de liquide entre en contact avec le premier côté du diaphragme ; et un générateur de vibrations capable de faire vibrer le diaphragme de manière à ce que le liquide rempli à l'intérieur du récipient de stockage de liquide soit atomisé sur le second côté du diaphragme par les ouvertures du diaphragme ;
ii) une chambre de mélange dans laquelle le générateur d'aérosol expulse ledit aérosol, la chambre de mélange étant en contact avec le second côté du diaphragme ;
iii) une valve d'inhalation qui est ouverte pour permettre une entrée d'air ambiant dans la chambre de mélange durant une phase d'inhalation et est fermée pour empêcher la fuite dudit aérosol de la chambre de mélange durant une phase d'expiration ; et
iv) une valve d'expiration qui est ouverte pour permettre la décharge de l'air respiré par un patient dans l'environnement durant la phase d'expiration et est fermée pour empêcher l'entrée d'air ambiant durant la phase d'inhalation ;
dans lequel la composition pharmaceutique est nébulisée par le nébuliseur d'inhalation pour former une composition aérosol et dans lequel au moins 90 % de l'inhibiteur de la protéinase alpha-1 pur et nébulisé se trouve sous sa forme active.

2. Système selon la revendication 1,
dans lequel l'inhibiteur de la protéinase alpha-1 purifié et stable est purifié d'un mélange non purifié de protéines par un procédé comprenant l'élution d'une pluralité de résines échangeuses d'ions, de préférence dans lequel l'inhibiteur de la protéinase alpha-1 purifié et stable est purifié d'un mélange non purifié de protéines par un procédé comprenant l'élution d'au moins deux résines échangeuses d'anions et d'au moins une résine échangeuse de cations ; ou
dans lequel le pH de la composition pharmaceutique se trouve dans la plage de 6,5 à 7,5 ; ou
dans lequel la concentration de l'inhibiteur de la protéinase alpha-1 purifié et stable est inférieure à 10 % p/v, de préférence dans lequel la concentration de l'inhibiteur de la protéinase alpha-1 purifié et stable est comprise entre 1 % et 5 %, de manière davantage préférée dans lequel la concentration de l'inhibiteur de la protéinase alpha-1 purifié et stable est d'environ 2 %.

3. Système selon la revendication 1,
dans lequel l'inhibiteur de la protéinase alpha-1 purifié et stable présente une pureté d'au moins 95 % ; ou
dans lequel l'inhibiteur de la protéinase alpha-1 purifié et stable présente une pureté d'au moins 99 %.

4. Système selon la revendication 1, dans lequel le diamètre médian massique des gouttelettes d'aérosol produites par le nébuliseur d'inhalation est inférieur à environ 5 µm ; ou
dans lequel le diamètre médian massique des gouttelettes d'aérosol est compris entre 1,0 µm et 4,0 µm ; ou
dans lequel le diamètre médian massique des gouttelettes d'aérosol est compris entre 2,0 µm et 3,5 µm ; ou
dans lequel le diamètre médian massique des gouttelettes d'aérosol est compris entre 2,5 µm et 3,3 µm.

5. Système selon la revendication 4, dans lequel l'écart type géométrique (ETG) est inférieur à environ 2,0 ; ou
dans lequel l'écart type géométrique (ETG) est inférieur à environ 1,7.

6. Système selon la revendication 1, dans lequel au moins 60 % de l'API sont dissous dans des gouttelettes d'aérosol inférieures à environ 5 µm.

7. Système selon la revendication 1, dans lequel la maladie pulmonaire est choisie dans le groupe constitué de l'emphysème ; d'une bronchopneumopathie chronique obstructive (BPCO) ; de la bronchiectasie ; de la tuberculose ; de maladies pulmonaires d'origine parenchymateuse ou fibrogène et de troubles comprenant la mucoviscidose, la fibrose pulmonaire interstitielle et la sarcoïdose ; et de maladies pulmonaires secondaires au VIH, de préférence
dans lequel l'emphysème est un emphysème héréditaire, ou de préférence dans lequel la maladie pulmonaire d'origine parenchymateuse ou fibrogène est la mucoviscidose.

8. Utilisation d'une (a) une composition pharmaceutique comprenant un inhibiteur de la protéinase alpha-1 (API) purifié, stable et actif produit par le foie et sécrété dans le système circulatoire sous forme de solution stérile prête à l'emploi, dans laquelle l'inhibiteur de la protéinase alpha-1 stable et purifié présente une pureté d'au moins 90 % ; et (b) un nébuliseur d'inhalation qui comprend :
i) un générateur d'aérosol qui comprend : un récipient de stockage de liquide qui comprend la composition pharmaceutique liquide ; un diaphragme ayant un premier côté et un second côté opposé ; le diaphragme ayant une pluralité d'ouvertures s'étendant à travers celui-ci, du premier côté au second côté, le premier côté étant connecté au récipient de stockage de liquide de telle sorte que le liquide rempli dans le récipient de stockage de liquide entre en contact avec le premier côté du diaphragme ; et un générateur de vibrations capable de faire vibrer le diaphragme de manière à ce que le liquide rempli à l'intérieur du récipient de stockage de liquide soit atomisé sur le second côté du diaphragme par les ouvertures du diaphragme ;
ii) une chambre de mélange dans laquelle le générateur d'aérosol expulse ledit aérosol, la chambre de mélange étant en contact avec le second côté du diaphragme ;
iii) une valve d'inhalation qui est ouverte pour permettre une entrée d'air ambiant dans la chambre de mélange durant une phase d'inhalation et est fermée pour empêcher la fuite dudit aérosol de la chambre de mélange durant une phase d'expiration ; et
iv) une valve d'expiration qui est ouverte pour permettre la décharge de l'air respiré par un patient dans l'environnement durant la phase d'expiration et est fermée pour empêcher l'entrée d'air ambiant durant la phase d'inhalation ;
pour la préparation d'un système comprenant ladite composition pharmaceutique et ledit nébuliseur, dans laquelle la composition pharmaceutique est nébulisée par le nébuliseur d'inhalation pour former une composition aérosol, pour l'administration à un sujet d'une quantité thérapeutiquement efficace de la composition aérosol comprenant un inhibiteur de la protéinase alpha-1, dans laquelle au moins 90 % de l'inhibiteur de la protéinase alpha-1 pur et nébulisé, se trouve sous sa forme active.

9. Utilisation selon la revendication 8, dans laquelle le système est destiné au traitement d'une maladie pulmonaire dans une phase d'exacerbation aiguë.

10. Utilisation selon l'une quelconque des revendications 8 à 9, dans laquelle la maladie pulmonaire est choisie dans le groupe constitué de l'emphysème ; d'une bronchopneumopathie chronique obstructive (BPCO) ; de la bronchiectasie ; de la tuberculose ; de maladies pulmonaires d'origine parenchymateuse ou fibrogène et de troubles comprenant la mucoviscidose, la fibrose pulmonaire interstitielle et la sarcoïdose ; et de maladies pulmonaires secondaires au VIH.

11. Utilisation selon la revendication 10,
dans laquelle l'emphysème est un emphysème héréditaire ; ou
dans laquelle la maladie pulmonaire d'origine parenchymateuse ou fibrogène est la mucoviscidose.

12. Utilisation selon la revendication 11,
dans laquelle au moins 50 % de l'API sont administrés au sujet ; ou
dans laquelle au moins 60 % de l'API sont administrés au sujet ; ou
dans laquelle au moins 70 % de l'API sont administrés au sujet.

13. Utilisation selon la revendication 12, dans laquelle l'API est absorbé par les tissus pulmonaires du sujet.

14. Utilisation selon la revendication 9, dans laquelle le sujet est un sujet humain.
